# EUROPEAN PATENT SPECIFICATION

(11) **EP 4 547 571 B1**
(45) Date of publication and mention of the grant of the patent: **20.05.2026**
(21) Application number: 23736101.9
(22) Date of filing: 28.06.2023
(51) Int. Cl.: B65D 41/04, A45D 34/04, B65D 51/24

(54) **PACKAGING DEVICE WITH CONTAINER WITH THREADED NECK AND SCREW CLOSURE MEMBER**
VERPACKUNGSVORRICHTUNG MIT BEHÄLTER MIT GEWINDEHALS UND SCHRAUBVERSCHLUSSGLIED
DISPOSITIF D'EMBALLAGE AVEC RÉCIPIENT À COL FILETÉ ET ÉLÉMENT DE FERMETURE À VIS

(30) Priority: 29.06.2022 FR 2206571
(43) Date of publication of application: 07.05.2025
(73) Proprietor: L'OREAL, 75008 Paris (FR)
(72) Inventor: LEROUX, Stéphane, 92110 CLICHY (FR)
(74) Representative: Ipsilon
(86) International application number: PCT/EP2023/067732
(87) International publication number: WO 2024/003189

(56) References cited:
- EP-B1- 0 816 244
- GB-A- 2 467 355
- US-A1- 2009 223 922
- US-A1- 2011 024 420
- US-A1- 2013 004 940
- US-A1- 2017 332 767
- US-B2- 8 091 724

## Description

### Technical field

The present invention relates to the field of devices for packaging a product, in particular a cosmetic product, having a container provided with a threaded neck, and a closure member that is to be screwed onto the neck.

### Prior art

In order to signal to the user that the closure member is screwed sufficiently, it is known to use signalling means that produce a tactile or audible "click".

To this end, US20200156831 and EP0780318 teach the use of closure members having a skirt provided with tongues that are capable of deforming elastically when they meet, at the end of screwing, protuberances provided on a shoulder of the container.

US8528759 and FR2572369 describe devices in which the "click" system, with or without tongue, and stop are situated at the end point of the thread, at the base of the neck and of the cap.

JP4509583 describes a device in which the "click" system and the stop are not situated on the threaded neck of the container but at another diameter of the container, different from that of the neck.

EP 0 816 244 describes an assembly comprising a neck of a container for soft drinks and a closure member to be screwed on the neck.

US 2013/004940 depicts a chamber having an opening for collecting a liquid sample and a lid for covering the opening.

US 2017/332767 describes a bottle comprising a body for containing a cosmetic product and a cover comprising a stem with an applicator tip.

GB 2 467 355, US 2009/223922, US 2011/024420,and US 8 091 724 describe a device for packaging a product according to the preamble of claim 1.

GB2203729 describes a container having a threaded neck on which is situated a protuberance and a cap having a thread interruption in which the protuberance is housed during the screwing of the cap onto the container, the protuberance being situated in a non-flexible zone.

US2008110850 discloses a container neck having a thread interruption in which a lug borne by a lid is engaged during the screwing of the lid onto the container, the passage of the lug over the thread of the neck causing deformation of the lid and/or of the neck. This device necessitates thin, flexible walls and is difficult to envisage in the case of rigid materials, such as glass.

In order to manufacture the one or more end-of-travel protuberances on the container, the method for manufacturing the container has to permit small radii of curvature so that the click is emitted with high quality. However, when the container is made of glass, the permitted radii of curvature are too large to allow the formation of such protuberances on the container in a satisfactory manner.

In addition, the presence of one or more end-of-travel protuberances on the shoulder of the container may detract from the attractiveness thereof. These protuberances can also promote the build-up of dirt, which is unattractive and could potentially lead to imperfect closure.

There is a need for a packaging device that is compatible with the production of the container from glass, if desired, allows the user to be informed, if necessary, of the correct closure of the container by generation of a click, and is relatively easy to produce and also attractive.

### Disclosure of the invention

The present invention meets this need by virtue of a device for packaging a product, in particular a cosmetic product, having:
- a container for containing the product, provided with a threaded neck having a longitudinal axis, at least one screw thread having, on the side of the free end of the neck, a thread starting point, and
- a closure member for closing the container that is arranged to be screwed onto the neck, the closure member having at least one corresponding stop arranged to come to bear against the thread starting point at the end of the screwing of the closure member onto the neck of the container to block the rotation of the closure member.

The device according to the invention makes it possible to block the screwing of the closure member without having to form a protuberance at the base of the neck.

The attractiveness of the container is thereby preserved, and it is possible to avoid the build-up of dirt at the base of the neck.

In addition, the container may be made of glass if desired.

### Container

The container may have a plurality of threads each having, on the side of the free end of the neck, a thread starting point.

In the following text, certain features of the thread are specified, it being understood that, in the presence of a plurality of threads, each of these threads may exhibit the features in question.

The thread starting point may have a stopping surface against which the corresponding stop comes to bear, this stopping surface being in particular substantially parallel to the longitudinal axis of the neck. As a variant, the stopping surface is inclined with respect to the longitudinal axis of the neck.

The screw thread may have, on the side opposite the free end of the neck, a thread end point, and may have a thread interruption between the thread starting point and the thread end point, which, by cooperating with a tongue of the closure member, makes it possible to generate a tactile and/or aural sensation informing the user of the correct closure of the container, as will be specified below.

The thread interruption may be situated between the thread starting point and the halfway point of the height of the thread.

The thread interruption may be angularly spaced apart from the thread starting point by less than 90° around the longitudinal axis of the neck, better still less than 45° around the longitudinal axis of the neck, better still less than 22.5° around the longitudinal axis of the neck.

The thread interruption may have an angular extent, around the longitudinal axis of the neck, of between 5° and 45°, in particular substantially equal to 30°.

The container has, at the base of the neck and around the latter, a shoulder, and the latter may not have a stop that forms a protrusion with respect to the shoulder and against which the closure member comes to bear at the end of the screwing thereof.

The absence of protrusion on the shoulder makes it possible to obtain an attractive container and to limit the risk of dirt build-up, as indicated above. Furthermore, the manufacture of such a container that does not have a protrusion on the shoulder is thus simplified.

The container may be made, at least in part, of glass. Even with a glass container, the invention makes it possible to obtain, at the end of screwing, clear and precise tactile or even also aural perception, affording the user high quality use of the device. The container can in particular be made, at least in part, of drawn glass. It may also be made, at least in part, of moulded glass. As a variant, the container may be made, at least in part, of a polymer, for example of a thermoplastic material.

The container can have a body of generally cylindrical, spherical, prismatic, in particular parallelepipedal, shape, among other possibilities.

The container may or may not be transparent.

The capacity of the container may be between 2 ml and 1000 ml.

The container can be filled with a product, in particular a cosmetic product, for example a mascara, a liquid foundation, a liquid lipstick, an anti-sun product, a hair or makeup-removal lotion, an anti-wrinkle product or a nail varnish, among other possibilities.

In an embodiment in which the container is intended to be filled with a makeup product, such as a mascara or a liquid lipstick, the capacity of the container is preferably between 2 ml and 15 ml, and in particular between 6 ml and 10 ml. It may then be in the form of a bottle combined with an applicator belonging to the closure member, the applicator comprising a gripping element intended to be handled by the user, an applicator member intended to be submerged inside the bottle so as to be loaded with product to be applied and a stem connecting the applicator member to the gripping element.

As a variant, the container may have a greater capacity, in particular of between 15 ml and 1000 ml, for example when it is intended to be filled with another type of cosmetic product, such as a liquid foundation, an anti-sun product, a care product or the like. The closure member may then have a dispensing system, such as a pump, a flip-top cap or the like, or a device for closing off the container, such as a stopper.

### Closure member

The closure member may have one or more stops arranged to come to bear against the one or more thread starting points at the end of the screwing of the closure member onto the neck of the container.

The or each stop may have a surface intended to come to bear against the thread starting point, this surface extending in particular substantially parallel to the longitudinal axis of the closure member. As a variant, this surface is inclined and extends in particular with the same inclination, relative to the longitudinal axis of the device, as the stopping surface of the thread starting point.

The closure member may have at least one elastically deformable tongue arranged to engage at least partially in the abovementioned thread interruption, during the screwing of the closure member onto the neck of the container, and generate a tactile sensation and/or the emission of an audible click.

Preferably, the angular extent of the tongue around the longitudinal axis of the closure member is greater than or equal to the angular extent of the thread interruption around the longitudinal axis of the neck.

Thus, the tongue may have an angular extent, around the longitudinal axis of the closure member, of between 10° and 55°, and in particular of the order of 30°.

The tongue may be attached to the closure member by only one of its ends. As a variant, the tongue may be attached to the closure member by both of its ends, then forming a bridge of material.

The tongue may have, on the inside, an upstream flank and a downstream flank that are oriented obliquely in the radial direction, the upstream flank engaging first in the thread interruption during the screwing of the closure member, and the downstream flank last, the upstream flank preferably having an inclination relative to the radial direction that is greater than that of the downstream flank. This asymmetric shape of the tongue makes it possible to make the passing of the tongue over the thread more difficult in the unscrewing direction than in the screwing direction. This may make it possible to reduce the risk of accidental opening of the container.

The tongue may in particular have, on its inner surface, at least one protruding relief, for example a boss, the relief being designed to engage in the thread interruption, the relief having an upstream flank and a downstream flank that are oriented obliquely in the radial direction, the upstream flank engaging first in the thread interruption during the screwing of the closure member, and the downstream flank last, the upstream flank preferably having an inclination relative to the radial direction that is greater than that of the downstream flank.

Preferably, the angular extent of the protruding relief, for example of the boss, around the longitudinal axis of the closure member is smaller than or equal to the angular extent of the thread interruption around the longitudinal axis of the neck.

Thus, the protruding relief may have an angular extent, around the longitudinal axis of the closure member, of between 3° and 45°, and in particular around 20°.

The closure member may have a cap arranged to be screwed onto the neck of the container, the cap being able to bear the tongue.

The closure member, in particular the cap, may have an internally threaded mounting skirt, arranged to be screwed onto the neck.

The mounting skirt may be provided with at least one perforation, the tongue being able to extend at least partially into this perforation.

The closure member may have an outer overcap that is disposed around the cap. This overcap may contribute to the attractiveness of the closure member, being for example made of metal or metallized.

The radial displacement of the tongue during the screwing or unscrewing may take place in the thickness of the wall of the cap.

In the presence of an overcap, the device may have, between the cap and the outer overcap, a space allowing the necessary radial displacement of the tongue, if appropriate.

The mounting skirt may have at least two angular skirt segments, as is described in application FR2113622, each of which is articulated with respect to an upper portion in the overall shape of a cylinder of revolution, between an initial, spaced-apart moulding position, in which the angular segments are oriented outwards away from the longitudinal axis of the closure member, and an assembled position, in which the angular segments are folded towards one another.

Advantageously, each of the angular segments comprises, on its internal surface, a thread portion forming a threaded surface in the assembled position.

The two angular segments can be moulded in the flat state without using a threaded core, and this considerably reduces the manufacturing time of the closure member, and makes it possible to reliably produce the threads. Furthermore, the moulding in the flat state of the angular skirt segments allows further freedom in terms of the design of this skirt, in particular in order to provide thereon protruding or recessed elements that could not be obtained if the skirt was annular and moulded using a threaded core.

Advantageously, each angular segment is connected to the upper portion by means of a film hinge.

The angular segments may be two in number, each of semicylindrical overall shape.

The mounting skirt may be provided with at least one perforation preceding the stop in the direction of screwing of the closure member onto the container.

The closure member may have a stem holder to which a stem bearing a member for applying the product is fastened, this stem being able to be inserted into the container when the closure member is screwed onto the container.

The applicator member may be of any type, for example brush, fine brush, flocked end piece, felt tip or capillary tip, inter alia.

### Device

The screwing of the closure member onto the container is done preferentially in less than one turn around the longitudinal axis of the neck, in particular in less than half a turn, or even than a third of a turn. As a variant, the screwing of the closure member onto the container can be done in more than one turn.

The neck may have a plurality of threads distributed angularly around the axis of the neck, in particular two threads that are symmetrical to one another. Each thread may have, on the side of the free end of the neck, a thread starting point and may have, on the opposite side, a thread end point with an interruption between the thread starting point and the thread end point.

The closure member may have two threads corresponding to the two threads of the neck, and may have two stops arranged to come to bear respectively against the two thread starting points of the neck at the end of the screwing of the closure member onto the neck of the container.

The closure member may also have two elastically deformable tongues, in particular diametrically opposite one another, each arranged to engage in an interruption of a corresponding thread of the neck during the screwing of the closure member onto the neck of the container.

### Manufacturing method

According to another of its aspects, the invention further relates to a method for manufacturing a packaging device according to the invention, as defined above, involving the following steps:
a) moulding, in the spaced-apart configuration, two generally semicylindrical angular skirt segments, which are each connected by a film hinge to an upper portion of the closure member,
b) after the moulding operation, folding the angular segments in the direction of one another so as to form a continuous generally cylindrical mounting skirt, centred on the longitudinal axis of the closure member.

The method can involve the step consisting in manufacturing the container, in particular from drawn glass, and mounting the closure member on the container.

As an alternative, the closure member may be obtained by an additive manufacturing technique, such as 3D printing.

### Brief description of the drawings

The invention may be better understood upon reading the following detailed description of non-limiting implementation examples thereof and upon studying the appended drawings, in which:
[Fig. 1] shows a schematic and partial view of an example of a device according to the invention,
[Fig. 2] shows a schematic and partial transverse cross section of the device in Figure 1,
[Fig. 3] is a schematic and partial view of the closure member,
[Fig. 4] shows, in schematic and partial longitudinal cross section, the closure member in Figure 3,
[Fig. 5] shows, in schematic and partial transverse cross section, the closure member in Figure 4,
[Fig. 6] shows a schematic and partial view of the container,
[Fig. 7] shows a schematic and partial view, from another viewing angle, of the container in Figure 6,
[Fig. 8A] to [Fig. 8C] illustrate the screwing of the closure member onto the container,
[Fig. 9A] shows a variant embodiment of the closure member,
[Fig. 9B] shows, in schematic and partial transverse cross section, the closure member in Figure 9A,
[Fig. 10] shows a variant embodiment of the closure member, and
[Fig. 11] shows a variant embodiment of the closure member.

### Detailed description

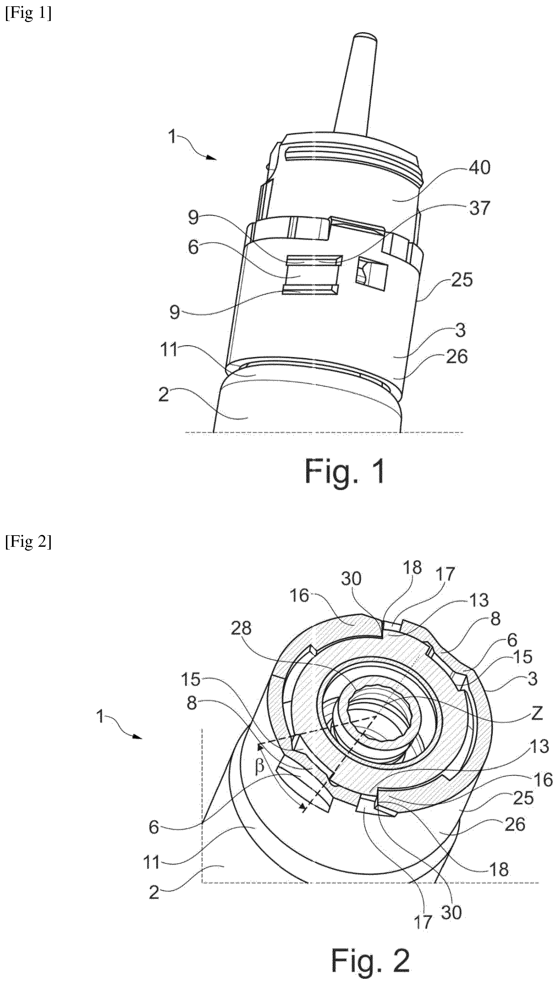

Figures 1 to 8C depict an example of a packaging device 1 according to the invention, having a container 2 and a closure member 3 arranged to be fastened to the container in order to close it.

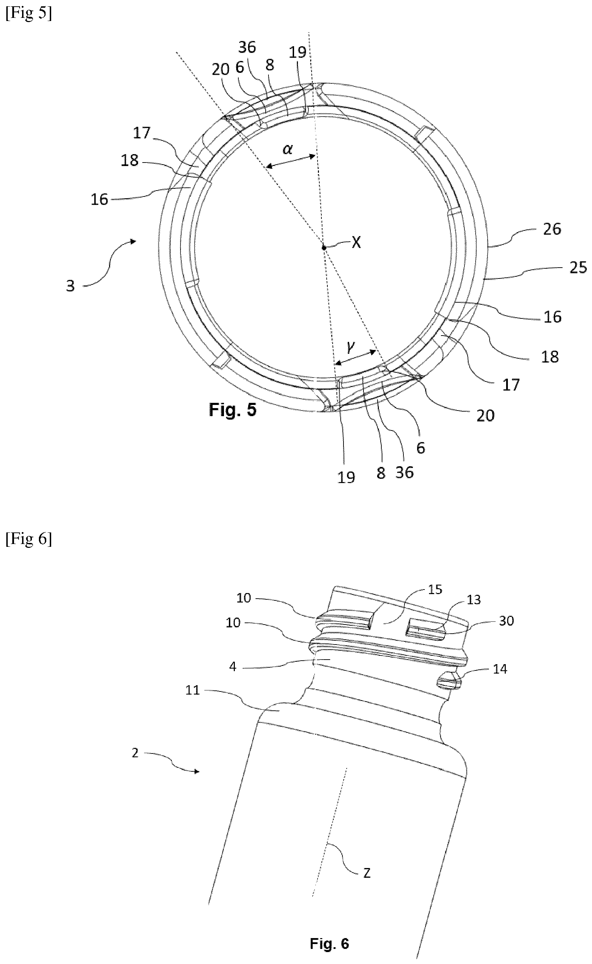

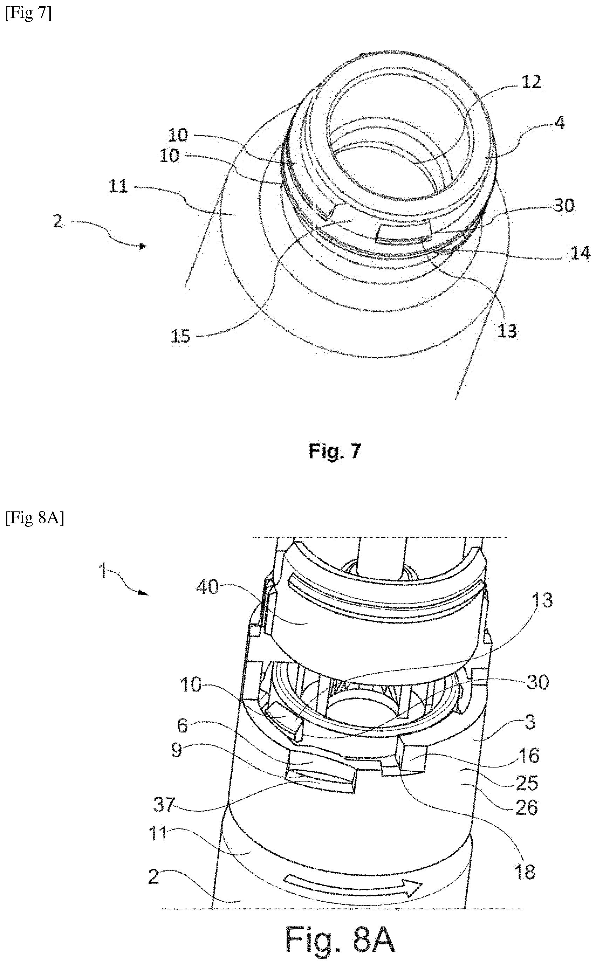

As can be seen in Figures 6 and 7, the container 2 has for example a body with a shape substantially in the form of a cylinder of revolution, of longitudinal axis Z, having at one end a shoulder 11 from which a threaded neck 4, onto which the closure member 3 is screwed, extends upwards.

The neck 4 has, in the example, two screw threads 10 that are diametrically opposite one another.

Each thread 10 may have a length and inclination such that the closure member 3 is screwed onto the container 2 in less than one turn.

The threads 10 each have a thread starting point 13 defining a stopping surface 30, this surface 30 extending substantially parallel to the longitudinal axis Z of the container 2. The threads 10 also each have a thread end point 14.

The container 2 is for example made of drawn glass, the neck 4 being integrally formed with the body of the container 2.

The container 2 contains for example a cosmetic product. Its capacity is for example between 2 and 15 ml.

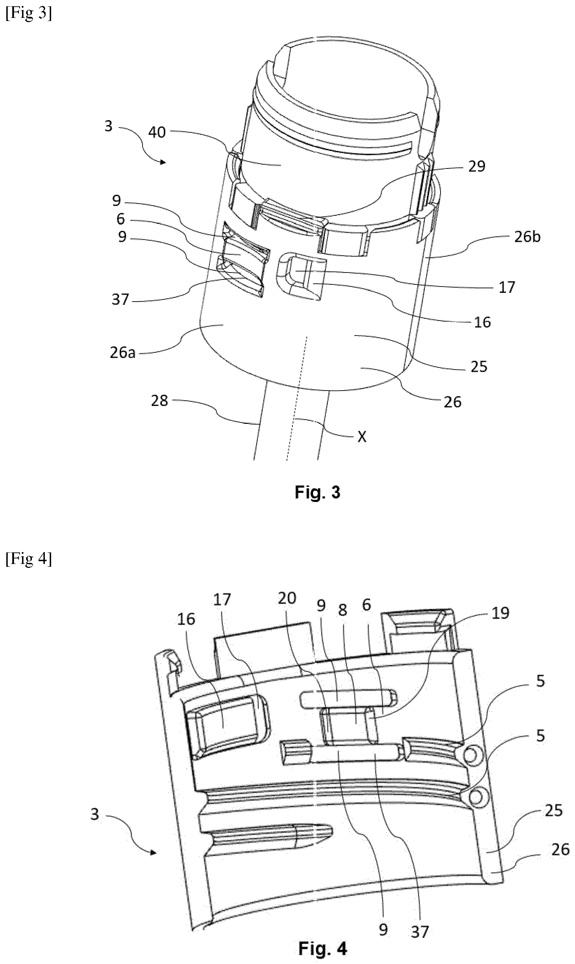

The closure member 3 has, as illustrated more particularly in Figures 3 to 5, a closure cap 25. The latter is made of a thermoplastic material.

The closure member 3 may also have an overcap, not shown, which makes it possible to give the closure member 3 the desired final appearance. This overcap may be metallic or metallized, among other possibilities.

The cap 25 has, in this example, an internally threaded tubular mounting skirt 26, comprising two threads 5 that are diametrically opposite one another cooperating respectively with the two threads 10 of the neck 4. As can be seen in Figure 3, the mounting skirt 26 has two angular skirt segments 26a and 26b, which are generally semicylindrical and diametrically opposite one another, forming the mounting skirt 26 when they are assembled, and each comprising, on its internal surface, a portion of the threads 5. These segments 26a and 26b are each connected by a film hinge 29 to an upper portion 40 of the closure member.

In the example in question, the cap 25 has two stops 16, each of the stops 16 being preceded, in the direction of screwing, by a perforation 17 created in the mounting skirt 26.

Each stop 16 has a surface 18 arranged to come to bear against one of the stopping surfaces 30 of the thread starting points 13, at the end of screwing of the cap 25 onto the neck 4.

Each surface 18 extends substantially parallel to the longitudinal axis X of the closure member 3.

The stops 16 and the stopping surfaces 30 are preferably positioned such that, at the end of the screwing, when they are bearing against one another, the lower edge of the cap 25 remains distant from the upper surface of the shoulder 11 by a non-zero distance; in other words, it is the stops 16 that limit the screwing of the cap 25 and not axial bearing of the closure member 3 against the shoulder 11 of the container 2.

The shoulder 11 can be produced with an attractive shape, exhibiting symmetry of revolution around the axis of the neck 4, without a protruding relief serving as end-of-screwing-travel stop, as can be seen in Figure 6.

The cap 25 may have, as illustrated, a stem 28, visible in Figure 3, bearing a member for applying the product (not shown), this stem 28 extending into the container 2 when the closure member 3 is screwed onto the container 2.

The latter may have a conventional wiping member (not shown), fastened in the opening 12 of the neck 4, the wiping member being able to cooperate with the stem 28 during unscrewing of the closure member 3 in order to wipe the stem 28 and eliminate, if appropriate, any surplus product from the application member.

In the example in question, the threads 10 have an interruption 15 between their starting point 13 and their end point 14. Each thread interruption 15 is for example, as illustrated, spaced apart by less than an eighth of a turn from the corresponding thread starting point 13.

Each thread interruption 15 may extend circumferentially around the longitudinal axis Z of the neck 4 over an angular extent β of the order of 30°.

The mounting skirt 26 may comprise, as can be seen in Figure 1 in particular, two flexible tongues 6, which are elastically deformable radially towards the outside and diametrically opposite one another, these tongues 6 each extending at least partially into a perforation 37 in the mounting skirt 26.

Each flexible tongue 6 may extend circumferentially around the longitudinal axis X of the closure member 3 over an angular extent α, which is preferably greater than or equal to the angular extent β of the thread interruption 15 around the longitudinal axis Z of the neck 4, for example of the order of 30°, being attached in this example by both of its circumferential ends to the mounting skirt 26, as illustrated in Figures 3 to 5 in particular.

The tongues 6 are inscribed, in the example in question, in the outer envelope surface in the form of a cylinder of revolution of the mounting skirt 26, a space 36 being provided in the thickness of the mounting skirt 26 so as to allow deformation thereof towards the outside without going beyond the cylindrical envelope of this mounting skirt 26.

Each tongue 6 forms a bridge of material extending between two slots 9, this bridge of material and these slots 9 having substantially the same inclination as the corresponding adjacent thread 5 formed protruding on the inner surface of the mounting skirt 26, as can be seen in Figure 4 in particular.

Each tongue 6 has a boss 8 that protrudes radially inwards, as can be seen in Figure 2 in particular, with a generally flattened apex connecting to an upstream flank 19 and a downstream flank 20 having a non-radial orientation.

Each boss 8 may extend circumferentially around the longitudinal axis X of the closure member 3 over an angular extent y, which is preferably smaller than or equal to the angular extent β of the thread interruption 15 around the longitudinal axis Z of the neck 4, for example of the order of 20°.

The tongues 6, and more specifically the bosses 8, are intended to engage in the corresponding thread interruptions 15 during the screwing of the closure member 3 onto the container 2, this making it possible to generate a tactile sensation, or even an audible click, informing the user of the correct closure of the device 1.

The upstream flank 19 engages first in the thread interruption 15, during the screwing of the closure member 3, and the downstream flank 20 last.

The upstream flank 19 preferably has an inclination, relative to the radial direction, greater than that of the downstream flank 20, and this makes it possible to generate the desired sensation and to make the passing of the tongue 6 over the thread 10 more difficult in the unscrewing direction than in the screwing direction, so as to reduce the risk of accidental opening of the container 2.

Figures 8A to 8C illustrate the screwing of the closure member 3 onto the container 2.

Figure 8A illustrates the closure member 3 during screwing onto the container 2. The tongue 6 is preparing to pass over the thread starting point 13.

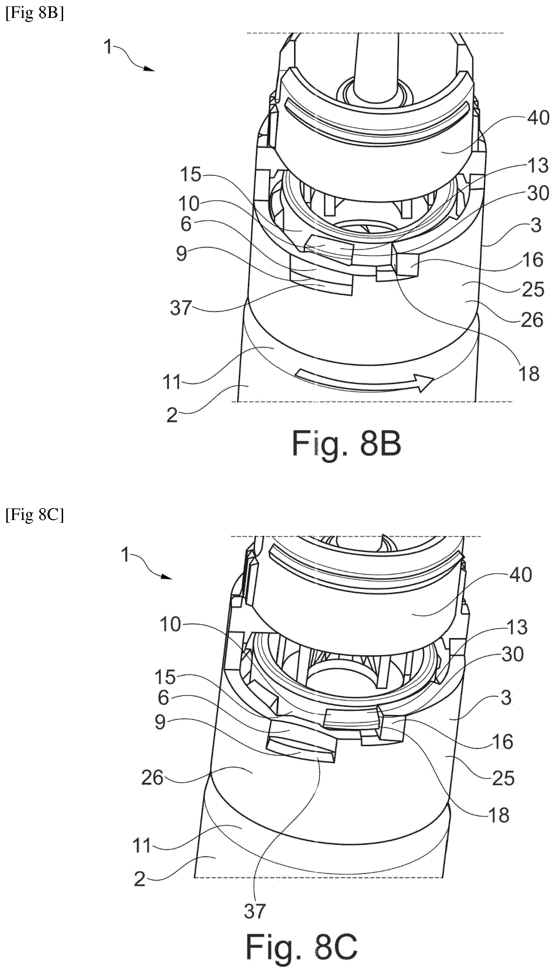

Figure 8B illustrates the passing of the tongue 6 over the thread starting point 13; in contact with the latter, the tongue 6 is deformed elastically radially outwards.

Figure 8C illustrates the end of screwing of the closure member 3 onto the container 2. The tongue 6 is then engaged in the thread interruption 15 and the surface 18 of the stop 16 comes to bear against the stopping surface 30 of the thread starting point 13.

The elastic return of the tongue 6 to its rest position, once entirely engaged in the thread interruption 15, may be perceived tactilely by the user and may be accompanied by an audible click. The engagement of the tongue 6 in the thread interruption 15 also makes it possible to help to keep the closure member 3 in the screwed position.

In order to manufacture the device 1, the cap 25 of the closure member 3 may be produced by injection moulding a thermoplastic material. The moulding can be done with the segments 26a and 26b in the spaced-apart position, these then being folded against one another by pivoting relative to the upper portion 40 by virtue of the film hinges 29. The segments 26a and 26b can be held against one another by the above-mentioned overcap.

It is possible, without departing from the scope of the present invention, to produce the tongues 6 in some other way.

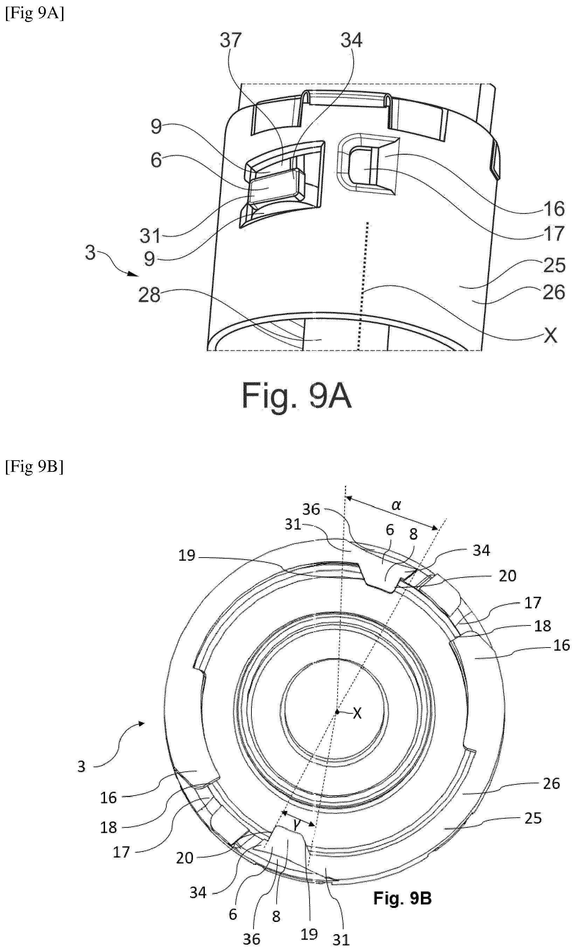

For example, as illustrated in Figures 9A and 9B, the tongue 6 may extend circumferentially around the longitudinal axis X of the closure member 3 and be attached to the mounting skirt 26 of the cap 25 only by an upstream end 31. "Upstream end of the tongue" should be understood to mean the end of the tongue 6 that engages first on the thread 10 of the neck 4 during the screwing of the closure member 3 onto the container 2. The opposite end 34 of the tongue 6, which is closest to the stop 16, is free.

The fact that the tongue 6 is produced with a free end makes it possible to increase the flexibility of the tongue 6 and make its radial deformation easier.

In Figure 9B, it can be seen that the downstream flank 20 may be oriented virtually radially, and this makes the end of passing over the thread portion 10 extending between the stopping surface 30 and the thread interruption 15 more abrupt, and therefore makes the tactile and/or audible sensation more marked.

In the example in Figures 9A and 9B, the tongue 6 is oriented in the direction of the screw thread 5. It is, however, possible to modify the orientation of the tongue 6 by attaching it to the mounting skirt 26 of the cap 25 in some other way, for example.

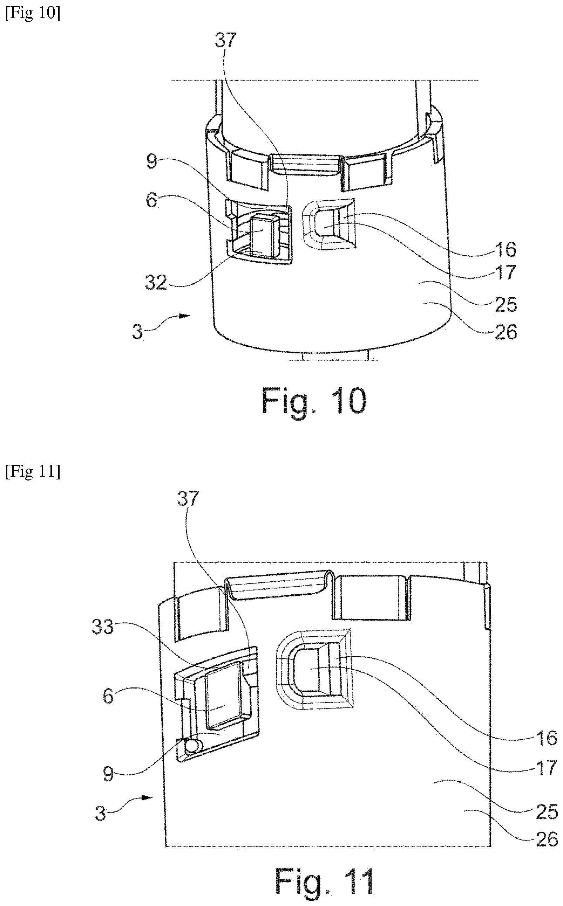

In the example in Figure 10, the tongue 6 extends axially, being attached to the mounting skirt 26 of the cap 25 by its lower end 32, and in the example of Figure 11 it also extends axially, but being attached to the mounting skirt 26 of the cap 25 by its upper end 33.

The number of tongues 6 may be different, as may the number of threads 5 or 10.

## Claims

1. Device (1) for packaging a product, in particular a cosmetic product, having:
- a container (2) for containing the product, provided with a threaded neck (4) having a longitudinal axis (Z), at least one screw thread (10) having, on the side of the free end of the neck, a thread starting point (13), and
- a closure member (3) for closing the container (2) that is arranged to be screwed onto the neck (4), the closure member (3) having at least one corresponding stop (16) arranged to come to bear against the thread starting point (13) at the end of the screwing of the closure member (2) onto the neck (4) of the container (2) to block the rotation of the closure member (3)
**characterized in that** at least one screw thread (10) has, on the side opposite the free end of the neck, a thread end point (14), and having a thread interruption (15) between the thread starting point (13) and the thread end point (14),
the closure member (3) having at least one elastically deformable tongue (6) arranged to engage at least partially in the thread interruption (15) during the screwing of the closure member (3) onto the neck (4) of the container (2) and generate a tactile sensation and/or the emission of an audible click.

2. Device according to the preceding claim, the thread starting point (13) having a stopping surface (30) against which the corresponding stop (16) comes to bear, this stopping surface (30) being substantially parallel to the longitudinal axis (Z) of the neck (4).

3. Device according to either of Claims 1 and 2, the thread interruption (15) being situated between the thread starting point (13) and the halfway point of the height of the thread (10).

4. Device according to any one of the preceding claims, the container (2) having, at the base of the neck (4) and around the latter, a shoulder (11), the latter not having a stop that forms a protrusion with respect to the shoulder (11) and against which the closure member (3) comes to bear at the end of the screwing thereof.

5. Device according to any one of the preceding claims, the container (2) being made, at least in part, of glass, in particular of drawn glass.

6. Device according to any one of the preceding claims, said at least one stop (16) having a surface (18) intended to come to bear against the thread starting point (13), this surface (18) extending substantially parallel to the longitudinal axis (X) of the closure member (3).

7. Device according to any one of the preceding claims, the tongue (6) being attached to the closure member (4) by only one of its ends (31; 32; 33).

8. Device according to any one of the preceding claims, the tongue (6) having, on the inside, an upstream flank (19) and a downstream flank (20) that are oriented obliquely in the radial direction, the upstream flank (19) engaging first in the thread interruption (15) during the screwing of the closure member (3), and the downstream flank (20) last, the upstream flank (19) preferably having an inclination relative to the radial direction that is greater than that of the downstream flank.

9. Device according to any one of the preceding claims, the closure member (3) having an internally threaded mounting skirt (26), arranged to be screwed onto the neck (4), provided with at least one perforation (37), the tongue (6) extending at least partially into this perforation (37).

10. Device according to any one of the preceding claims, the closure member (3) having an internally threaded mounting skirt (26), arranged to be screwed onto the neck (4), provided with at least one perforation (17), this perforation (17) preceding the stop (16) in the direction of screwing of the closure member (3) onto the container (2).

11. Device according to any one of the preceding claims, the closure member (3) having a stem holder to which a stem (28) bearing a member for applying the product is fastened, the stem (28) being inserted into the container (2) when the closure member (3) is screwed onto the container (2).

12. Device according to any one of the preceding claims, the screwing of the closure member (3) onto the container (2) being done in less than one turn around the longitudinal axis (Z) of the neck (4).

## Patentansprüche

1. Vorrichtung (1) zum Verpacken eines Produkts, insbesondere eines kosmetischen Produkts, aufweisend:
- einen Behälter (2) zur Aufnahme des Produkts, der mit einem Gewindehals (4) mit einer Längsachse (Z) und mindestens einem Schraubgewinde (10) mit einem Gewindeanfangspunkt (13) seitlich des freien Endes des Halses versehen ist, und
- ein Verschlussglied (3) zum Verschließen des Behälters (2), das zum Aufschrauben auf den Hals (4) angeordnet ist, wobei das Verschlussglied (3) mindestens einen entsprechenden Anschlag (16) aufweist, der so angeordnet ist, dass er am Ende des Aufschraubens des Verschlussglieds (2) auf den Hals (4) des Behälters (2) zur Anlage an den Gewindeanfangspunkt (13) kommt, um die Drehung des Verschlussglieds (3) zu blockieren,
**dadurch gekennzeichnet, dass** mindestens ein Schraubgewinde (10) auf der dem freien Ende des Halses gegenüberliegenden Seite einen Gewindeendpunkt (14) aufweist und eine Gewindeunterbrechung (15) zwischen dem Gewindeanfangspunkt (13) und dem Gewindeendpunkt (14) aufweist,
wobei das Verschlussglied (3) mindestens eine elastisch verformbare Zunge (6) aufweist, die so angeordnet ist, dass sie beim Aufschrauben des Verschlussglieds (3) auf den Hals (4) des Behälters (2) die Gewindeunterbrechung (15) mindestens teilweise in Eingriff nimmt und eine taktile Wahrnehmung und/oder die Abgabe eines hörbaren Klickens erzeugt.

2. Vorrichtung nach dem vorhergehenden Anspruch, wobei der Gewindeanfangspunkt (13) eine Anschlagfläche (30) aufweist, an der der entsprechende Anschlag (16) zur Anlage kommt, wobei diese Anschlagfläche (30) im Wesentlichen parallel zu der Längsachse (Z) des Halses (4) verläuft.

3. Vorrichtung nach einem der Ansprüche 1 oder 2, wobei die Gewindeunterbrechung (15) zwischen dem Gewindeanfangspunkt (13) und auf halber Höhe des Gewindes (10) liegt.

4. Vorrichtung nach einem der vorhergehenden Ansprüche, wobei der Behälter (2) an der Basis des Halses (4) und um diesen herum einen Absatz (11) aufweist, wobei Letzterer keinen Anschlag aufweist, der einen Vorsprung bezüglich des Absatzes (11) bildet und an dem das Verschlussglied (3) am Ende seines Aufschraubens zur Anlage kommt.

5. Vorrichtung nach einem der vorhergehenden Ansprüche, wobei der Behälter (2) zumindest teilweise aus Glas, insbesondere aus gezogenem Glas, hergestellt ist.

6. Vorrichtung nach einem der vorhergehenden Ansprüche, wobei der mindestens eine Anschlag (16) eine Fläche (18) aufweist, die dazu bestimmt ist, an dem Gewindeanfangspunkt (13) zur Anlage zu kommen, wobei sich diese Fläche (18) im Wesentlichen parallel zu der Längsachse (X) des Verschlussglieds (3) erstreckt.

7. Vorrichtung nach einem der vorhergehenden Ansprüche, wobei die Zunge (6) nur mit einem ihrer Enden (31; 32; 33) an dem Verschlussglied (4) angebracht ist.

8. Vorrichtung nach einem der vorhergehenden Ansprüche, wobei die Zunge (6) innenseitig eine stromaufwärtige Flanke (19) und eine stromabwärtige Flanke (20) aufweist, die in radialer Richtung schräg ausgerichtet sind, wobei die stromaufwärtige Flanke (19) beim Aufschrauben des Verschlussglieds (3) zuerst in der Gewindeunterbrechung (15) in Eingriff kommt und die stromabwärtige Flanke (20) zuletzt, wobei die stromaufwärtige Flanke (19) vorzugsweise eine Neigung bezüglich der radialen Richtung hat, die größer als die der stromabwärtigen Flanke ist.

9. Vorrichtung nach einem der vorhergehenden Ansprüche, wobei das Verschlussglied (3) einen mit einem Innengewinde versehenen Montagekragen (26) aufweist, der dazu angeordnet ist, auf den Hals (4) aufgeschraubt zu werden und mit mindestens einer Perforation (37) versehen ist, wobei sich die Zunge (6) mindestens teilweise in diese Perforation (37) erstreckt.

10. Vorrichtung nach einem der vorhergehenden Ansprüche, wobei das Verschlussglied (3) einen mit einem Innengewinde versehenen Montagekragen (26) aufweist, der dazu angeordnet ist, auf den Hals (4) aufgeschraubt zu werden und mit mindestens einer Perforation (17) versehen ist, wobei diese Perforation (17) dem Anschlag (16) in Richtung des Aufschraubens des Verschlussglieds (3) auf den Behälter (2) vorausgeht.

11. Vorrichtung nach einem der vorhergehenden Ansprüche, wobei das Verschlussglied (3) einen Stielhalter aufweist, an dem ein ein Glied zum Aufbringen des Produkts tragender Stiel (28) befestigt ist, wobei der Stiel (28) beim Aufschrauben des Verschlussglieds (3) auf den Behälter (2) in den Behälter (2) eingeführt wird.

12. Vorrichtung nach einem der vorhergehenden Ansprüche, wobei das Aufschrauben des Verschlussglieds (3) auf den Behälter (2) in weniger als einer Umdrehung um die Längsachse (Z) des Halses (4) erfolgt.

## Revendications

1. Dispositif (1) de conditionnement d'un produit, notamment un produit cosmétique, comportant :
- un récipient (2) pour contenir le produit, pourvu d'un col fileté (4) d'axe longitudinal (Z), au moins un filet (10) présentant, du côté de l'extrémité libre du col, une entrée de filet (13), et
- un élément de fermeture (3) pour fermer le récipient (2) qui est conçu pour se visser sur le col (4), l'élément de fermeture (3) comportant au moins une butée (16) correspondante conçue pour venir en appui contre l'entrée de filet (13) à la fin du vissage de l'élément de fermeture (2) sur le col (4) du récipient (2) pour bloquer la rotation de l'élément de fermeture (3)
**caractérisé en ce qu'**au moins un filet (10) présente, du côté opposé à l'extrémité libre du col, une fin de filet (14), et présente une interruption de filet (15) entre l'entrée de filet (13) et la fin de filet (14),
l'élément de fermeture (3) comportant au moins une languette élastiquement déformable (6) conçue pour entrer au moins partiellement en prise dans l'interruption de filet (15) lors du vissage de l'élément de fermeture (3) sur le col (4) du récipient (2) et produire une sensation tactile et/ou l'émission d'un clic sonore.

2. Dispositif selon la revendication précédente, l'entrée de filet (13) présentant une surface d'arrêt (30) contre laquelle vient en appui la butée (16) correspondante, cette surface d'arrêt (30) étant sensiblement parallèle à l'axe longitudinal (Z) du col (4).

3. Dispositif selon l'une ou l'autre des revendications 1 et 2, l'interruption de filet (15) étant située entre l'entrée de filet (13) et le point de mi-hauteur du filet (10).

4. Dispositif selon l'une quelconque des revendications précédentes, le récipient (2) comportant, à la base du col (4) et autour de celui-ci, un épaulement (11), celui-ci ne présentant pas de butée formant saillie par rapport à l'épaulement (11) et contre laquelle l'élément de fermeture (3) vient en appui à la fin de son vissage.

5. Dispositif selon l'une quelconque des revendications précédentes, le récipient (2) étant réalisé, au moins en partie, en verre, notamment en verre étiré.

6. Dispositif selon l'une quelconque des revendications précédentes, ladite au moins une butée (16) comportant une surface (18) destinée à venir en appui contre l'entrée de filet (13), cette surface (18) s'étendant sensiblement parallèlement à l'axe longitudinal (X) de l'élément de fermeture (3).

7. Dispositif selon l'une quelconque des revendications précédentes, la languette (6) étant fixée à l'élément de fermeture (4) par une seule de ses extrémités (31 ; 32 ; 33).

8. Dispositif selon l'une quelconque des revendications précédentes, la languette (6) présentant, du côté intérieur, un flanc amont (19) et un flanc aval (20) orientés obliquement dans la direction radiale, le flanc amont (19) entrant en premier en prise dans l'interruption de filet (15) lors du vissage de l'élément de fermeture (3), et le flanc aval (20) en dernier, le flanc amont (19) présentant de préférence une inclinaison par rapport à la direction radiale plus grande que celle du flanc aval.

9. Dispositif selon l'une quelconque des revendications précédentes, l'élément de fermeture (3) comportant une jupe de montage (26) filetée intérieurement, conçue pour se visser sur le col (4), pourvue d'au moins une perforation (37), la languette (6) s'étendant au moins partiellement dans cette perforation (37).

10. Dispositif selon l'une quelconque des revendications précédentes, l'élément de fermeture (3) comportant une jupe de montage (26) filetée intérieurement, conçue pour se visser sur le col (4), pourvue d'au moins une perforation (17), cette perforation (17) précédant la butée (16) dans le sens de vissage de l'élément de fermeture (3) sur le récipient (2).

11. Dispositif selon l'une quelconque des revendications précédentes, l'élément de fermeture (3) comportant un support de tige sur lequel est fixée une tige (28) portant un élément d'application du produit, la tige (28) étant insérée dans le récipient (2) lorsque l'élément de fermeture (3) est vissé sur le récipient (2).

12. Dispositif selon l'une quelconque des revendications précédentes, le vissage de l'élément de fermeture (3) sur le récipient (2) se faisant en moins d'un tour autour de l'axe longitudinal (Z) du col (4).
